# EUROPEAN PATENT APPLICATION

(11) **EP 3 811 880 A1**
(43) Date of publication of application: **28.04.2021**
(21) Application number: 19822729.0
(22) Date of filing: 06.06.2019
(51) Int. Cl.: A61B 17/3211

(54) **SCALPEL FOR CARPAL TUNNEL SURGERY**

(30) Priority: 19.06.2018 ES 201830943 U
(71) Applicant: Com-medy Health Devices S.L., 46008 Valencia (ES)
(72) Inventor: COLOMA SAIZ, Javier, 46004 Valencia (ES)
(74) Representative: Escamilla Condés, Mónica
(86) International application number: PCT/ES2019/000040
(87) International publication number: WO 2019/243643

(57) **Abstract**

Scalpel for carpal tunnel surgery by sectioning the transverse carpal ligament; comprising: - a handle (1), - an actuator (2) housed in the handle (1) with the possibility of longitudinal movement, - a hollow anterior guide (4) attached to the front end of the handle and protruding frontally from the handle (1), comprising the said anterior guide (4) a longitudinal slot (41), and a hook (42) at its free end aligned with the aforementioned longitudinal slot (41), and suitable for the attachment of the transverse carpal ligament to be sectioned and; - a knife (5) for sectioning the transverse carpal ligament, being the knife (5) attached to the actuator (2) by a shaft (51), and with the possibility to move along the longitudinal slot (41) of the anterior guide (4) from an inoperative rear position to an operational anterior cutting position, limited by the hook (42).

## Description

### Field of application

The object of the present report is a scalpel, applicable in the surgical sector, specially designed to perform a minimally invasive intervention on an outpatient basis for the treatment of carpal tunnel syndrome by sectioning the transverse carpal ligament, speeding up the procedure and avoiding the need for the patient to go through an operating room, with the resulting comfort for him/her and the cost savings for hospitals and health systems and/or private insurers.

### State of the art

At present, the definitive treatment of carpal tunnel syndrome requires that the patient be hospitalised and treated in the operating room, which implies a high economic cost and significant management of resources (hospital bed, qualified personnel, etc.).

The high prevalence of carpal tunnel syndrome and the long waiting lists for surgery mean a longer time of symptomatology and absence from work for the patient until the surgery is performed.

On the other hand, the surgical technique traditionally used, that is, the technique of open surgery implies that a longitudinal incision is made in the palm of the patient's hand, which ultimately requires more postoperative medical care, sick leave, and most of the time, a rather painful recovery.

On the other hand, the endoscopic techniques used to a lesser extent require a large number of resources (camera, television, special scalpels adapted to this type of techniques, etc.) that make the investment and economic return difficult to achieve in the public sphere, such that their employment is virtually reduced to some private centres.

### Description of the invention

The technical problem that is resolved by the present invention is to obtain a tool of simple use for the orthopaedic surgeon, capable of performing a minimally invasive surgery through a small transverse incision on the volar face of the wrist. It allows conversion of carpal tunnel surgery into an outpatient intervention, with the resulting economic savings and improvements in the recovery quality of the patients (referred to postoperative).

For this purpose, the surgical scalpel for the carpal tunnel, the object of this invention, comprises: - a handle, - an actuator housed in the handle with the possibility of longitudinal movement, - an anterior guide, hollow, fixed to the anterior end of the handle and protruding frontally from the handle, comprising said anterior guide of a longitudinal slot, with a hook at its free end aligned with the aforementioned slot and suitable for the attachment of the transverse carpal ligament to be sectioned and, - a knife for sectioning the transverse carpal ligament, being said knife attached to the actuator by an axis, and with possible movement along the anterior guide slot between an inoperative posterior position and an operational anterior cutting position limited by the hook.

In a first embodiment, the handle has a threaded end for its union with a front stop that incorporates the aforementioned hollow anterior guide in its central part. The rear guide includes a bushing in its central part at its distal end that acts as a means of joining the knife shaft of the scalpel, using hardware or equivalent.

Due to its design as explained above, the scalpel can be used on an outpatient basis (in external consultations) without the need for an operating room or more expensive endoscopic interventions, permitting the patient to avoid the procedure of going to the operating room (waiting days, pre-anaesthesia, anaesthesia, or sedation in the operating room), and the hospital to avoid the costs of surgery.

This scalpel allows the operation to be performed percutaneously through a small transverse incision in the volar face of the wrist and, consequently, an earlier return of the patient to work. In this manner, scars are avoided on the palm of the hand. It also eliminates the risk of accidental damage to other structures, thanks to the metal guide and the limit provided by the hook. Another advantage is precisely the hook because it allows the surgeon to know at all times the distal limit of the ligament, since the ligament is "hooked" by the hook of the anterior guide and can even be felt in the palm of the hand. This anterior guide has laser markings that advise how far the anterior guide is inserted through the incision.

The "classical" carpal tunnel operation performed with the "Taleisnik approach" (longitudinal incision in the palm of the hand, over the annular ligament), results in a lot of postoperative discomfort due to the large number of sensitive nerve endings in the skin around this area. Therefore, there is a high risk of cutting these nerve endings, often creating very annoying and painful scars. With the scalpel recommended herein, a small transverse incision in the wrist will avoid this problem.

Furthermore, for this type of "classic" approach, the wound in this anatomical area takes about three weeks to heal, and dehiscences of the sutures can occur resulting from sweat and traction. This problem is avoided by using the scalpel presented herein.

### Brief description of the figures.

The following briefly describes a series of drawings that help to understand the invention better and that are expressly related to an embodiment of the said invention that is presented as a non-limiting example of it.
- Fig. 1 shows a side view of a first embodiment of the scalpel for carpal tunnel surgery, the object of this invention.
- Fig. 2 shows a plan view of the carpal tunnel surgical scalpel in Figure 1.
- Fig. 3 shows a view of the handle (1) as part of the carpal tunnel surgical scalpel in Figure 1.
- Fig. 4 shows a view of the actuator (2) as part of the carpal tunnel surgical scalpel in Figure 1.
- Fig. 5 shows a view of the anterior guide (4) as part of the carpal tunnel surgical scalpel in Figure 1.
- Fig. 6 shows a view of the knife (5) as part of the carpal tunnel surgical scalpel in Figure 1.
- Fig. 7 shows a perspective view of a second embodiment of the carpal tunnel surgical scalpel, according to the invention.
- Fig. 8 shows an elevational view of the scalpel in Figure 7.
- Fig. 9 shows an elevational view of the scalpel in Figures 7 and 8, partially sectioned by a vertical plane and with the knife in an inoperative position.
- Fig. 10 shows an elevational view of the scalpel in Figures 7 and 8, partially sectioned by a vertical plane and with the knife in the operating position.
- Fig. 11 shows a cross-section of the scalpel in the A-A plane referenced in Figure 9.
- Fig. 12 shows a cross-section of the scalpel in the B-B plane referenced in Figure 9.

### Detailed description of the embodiment modes of the invention.

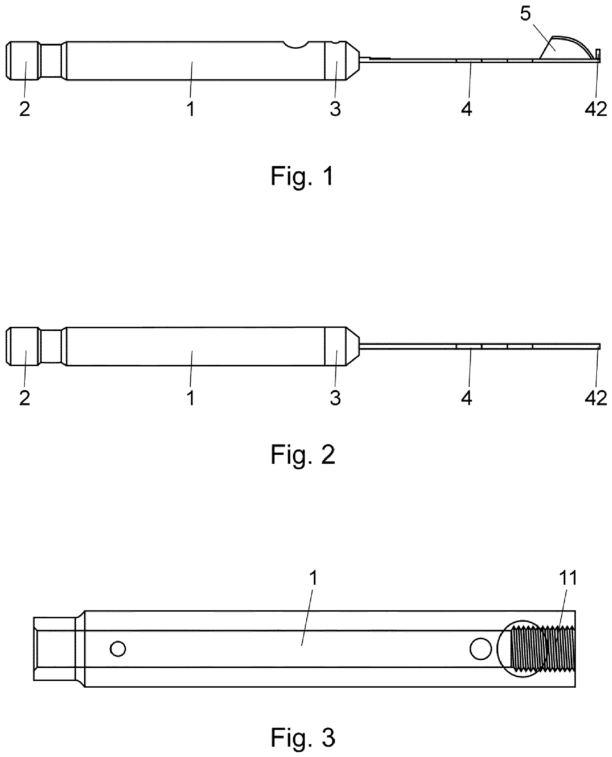

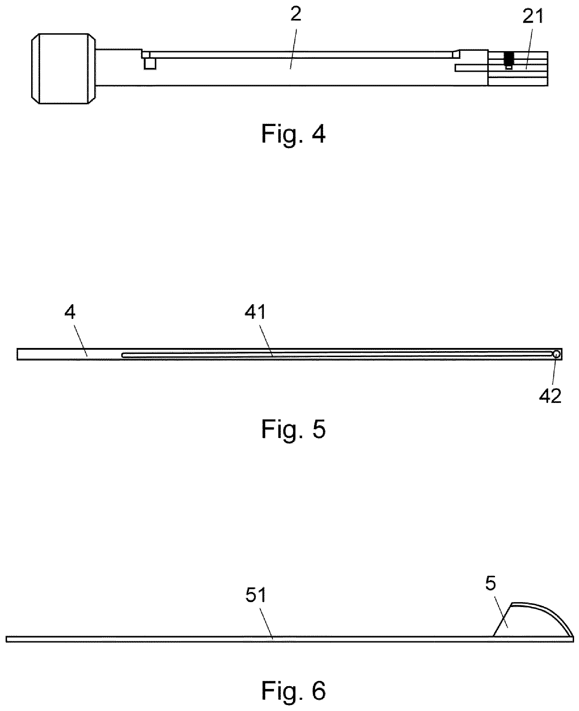

Figures 1 to 6 show a first embodiment of the scalpel for carpal tunnel surgery, the object of this invention.

In this first embodiment, the scalpel comprises a handle (1) wherein an actuator (2) is fitted with the possibility of longitudinal travel.

The handle has a threaded end (11) wherein a front stop (3) is attached solidly conforming to the front end of the handle (1).

The actuator (2) incorporates a bushing (21) at its distal part, which acts as a means of joining the knife shaft (51) of the scalpel (5), using hardware or equivalent.

The front stop (3) incorporates in its central part a hollow, solid anterior guide (4) protruding from the front of the handle (1); comprising this anterior guide (4) a longitudinal slot (41) and, at its free end, a hook (42) that is aligned with the aforementioned longitudinal slot (41), and suitable for attachment of the transverse carpal ligament to be sectioned.

This anterior guide (4) has transverse laser markings, advising the degree of penetration of the anterior guide in the patient during the procedure.

This hollow anterior guide (4) is occupied in its interior by the shaft (51) of the knife (5).

The longitudinal slot (41) of the anterior guide (4) allows the longitudinal movement of the knife (5), responsible for sectioning the ligament, from an inoperative posterior position to an anterior operational position for cutting the ligament, limited by the hook (42).

The shaft (51) is attached to the actuator (2) at the opposite end of the knife (5), allowing the knife (5) to be moved along the longitudinal slot (41) of the anterior guide (4), in the proximal or distal direction and without the possibility of a change of direction when actuating the actuator (2) in either direction.

In the first embodiment of the invention, the handle (1), the actuator (2), and the front stop (3) are preferably made of plastic, while the other components will be made of stainless steel or a material with equivalent mechanical characteristics, all of which are sterilisable and suitable for use in health care.

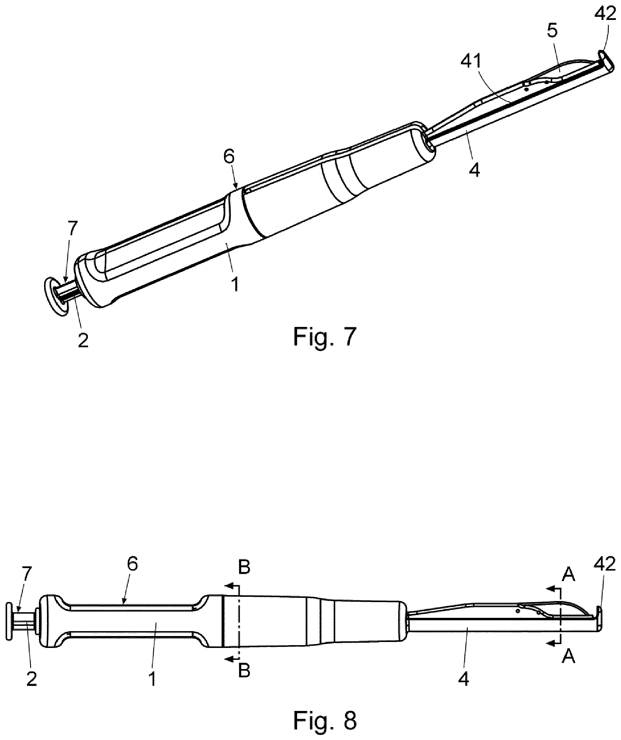

In the second embodiment, shown in figures 7 to 12, the scalpel maintains the above-described operation and presents a greater simplicity of construction.

Specifically, in this second embodiment, the handle (1) and the anterior guide (4) are formed by one piece (6), which in this embodiment consists of two halves (61, 62), that permit assembly in its interior of the actuator (2) and the knife shaft (51), prior to the attachment of these halves (61, 62) by welding, screwing, or equivalent.

The part (6) is made in this case with a plastic material for surgical use, which allows the significant reduction of manufacturing costs compared to scalpels made entirely of metal, although the use of other materials suitable for surgical use is not excluded.

In this second embodiment, the knife (5) shaft (51) and actuator (2) are made of a single part (7), preferably of plastic material for surgical use, such that the knife (5) is mounted in a housing (71) defined at the front end of the shaft (51).

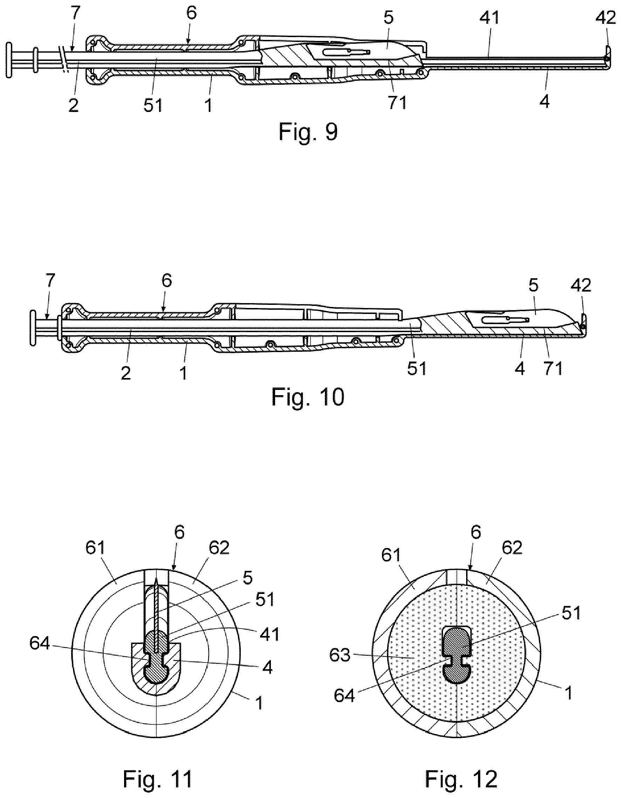

As can be seen in Figures 11 and 12, the part (6) presents in the longitudinal slot (41) of the anterior guide (4) and some transverse partitions (63) of the handle (1), some non-circular sections (64), suitable for longitudinal guidance, and without the possibility of rotation of the knife shaft (51) (5) and the actuator (2).

## Claims

1. Scalpel for carpal tunnel surgery, specifically through the sectioning of the carpal transversal ligament **which is characterised in that** it comprises:
- a handle (1),
- an actuator (2) housed in the handle (1) with the possibility of longitudinal movement,
- a hollow anterior guide (4) attached to the anterior end of the handle and protruding frontally from the handle (1), comprising the anterior guide (4) a longitudinal slot (41), and a hook (42) at its free end aligned with the aforementioned longitudinal slot (41), and suitable for holding the transverse carpal ligament to be sectioned, and;
- a knife (5) for sectioning the transverse carpal ligament, being this knife (5) attached to the actuator (2) by a shaft (51), and with the possibility to move along the longitudinal slot (41) of the anterior guide (4), from an inoperative rear position to an operational anterior cutting position, limited by the hook (42).

2. Scalpel, according to Claim 1, **characterised in that** the handle (1) presents one of its ends (11) threaded for its union with a front stop (3) that incorporates the hollow anterior guide (4) in its central part.

3. Scalpel, according to Claim 1, **characterised in that** the actuator (2) incorporates in its distal part a bushing (21) which serves as a means of joining with the shaft (51) of the knife (5), utilising hardware or equivalent.

4. Scalpel, according to any of the previous claims, **characterised in that** the handle (1), the actuator (2) and the front stop (3) are preferably made of plastic while the rest of the components, are either made of stainless steel or a material with equivalent mechanical characteristics, all of which are sterilisable and suitable for use in health care.

5. Scalpel, according to Claim 1, **characterised in that** the handle (1) and the anterior guide (4) are made of a single piece (6) or in two halves (61, 62) of that piece (6).

6. Scalpel, according to Claim 5, **characterised in that** the piece (6) is made of plastic material for surgical use.

7. Scalpel, according to Claim 5, **characterised in that** the shaft (51) of the knife (5) and the actuator (2) are made of one piece (7), being mounted this knife (5) in a housing (71) defined on the anterior end of shaft (51).

8. Scalpel, according to Claim 7, **characterised in that** the piece (7) is made of plastic material for surgical use.

9. Scalpel, according to Claim 5, **characterised in that** the piece (6) presents in the longitudinal slot (41) of the anterior guide (4), and some transverse partitions (63) of the handle (1), some non-circular sections (64), suitable for longitudinal guidance, and without the possibility of rotation of the knife shaft (51) (5) and the actuator (2).
